# EUROPEAN PATENT APPLICATION

(11) **EP 1 114 867 A1**
(43) Date of publication of application: **11.07.2001**
(21) Application number: 99126004.3
(22) Date of filing: 27.12.1999
(51) Int. Cl.: C12N 15/82, C07K 14/415, C12N 1/13, A01H 3/00, C12R 1/89, C12N 1/12

(54) **Transcription regulatory sequences derived from Chlamydomonas reinhardtii**

(71) Applicant: UNIVERSITE DE GENEVE, CH-1211 Geneve 4 (CH)
(72) Inventor: Fischer, Nicolas, 1205 Geneve (CH); Rochaix, Jean-David, 1295 Mies (CH)
(74) Representative: Almond-Martin, Carol

(57) **Abstract**

The invention concerns a transcription regulatory element, characterised in that it comprises :
i) the nucleic acid sequence illustrated in Figure 7, or
ii) a fragment thereof, or
iii) a sequence having at least 70% identity with the sequence of Figure 7.

## Description

The present invention relates to transcription regulatory sequences derived from the green alga *Chlamydomonas reinhardtii,* particularly from the psaD gene of this organism. The invention also relates to the use of these regulatory sequences in chimeric genes for the nuclear transformation of eukaryotic cells such as higher plants and green algae. The transcription regulatory sequences of the invention permit high level expression of endogenous and exogenous nucleic acids in eukaryotic organisms belonging to the phylogenetic kingdom *Planta*.

In recent years, the unicellular green alga *Chlamydomonas reinhardti* has emerged as a powerful model system for basic and applied research. Major technical advances have greatly increased the interest for this organism. In particular, reliable transformation methods for the chloroplast, mitochondrial and nuclear genomes are available (Rochaix, 1995). Gene tagging strategies and rescue of mutants with genomic libraries are feasible for the discovery of new genes. Furthermore, its short generation time can be exploited to rapidly test novel strategies that can be later applied to the field of plant molecular biology and crops development. It is also possible that Chlamydomonas may be used as a host for the production of recombinant proteins.

However, expression of heterologous nuclear genes is not easy to achieve in *Chlamydomonas*. Even reporter genes which are routinely expressed in other eukaryotic systems have been found to be problematic (Day et al, 1990, Blankenship and Kindle, 1992, Kindle and Sodeinde 1994).

The reasons for the lack of expression have not been entirely elucidated. They could include position effects, inefficient transcription from heterologous promoters, gene silencing, inaccurate RNA processing or export from the nucleus and mRNA instability. It is also possible that defective translation of foreign sequences not matching the biased codon usage of Chlamydomonas adversely affects expression. Indeed, most *Chlamydomonas* nuclear genes feature a pronounced codon bias towards G or C, which is reflected in the high GC content of the nuclear genome.

A further potential difficulty may arise from the absence of intronic sequences in cDNAs, often used in transformation. Indeed, another general characteristic of nuclear genes in Chlamydomonas is the presence of introns that are often required for gene expression and regulation. Although some intronless genes have been described, intronic sequences appear to play an important role for efficient gene expression (Silflow, 1998). It has recently been clearly demonstrated that the insertion of an intron of *rbcS* into the Ble gene dramatically increased the expression levels from the *rbcS* promoter (Lumbreras et al., 1998). The improvement was found to be mediated in part by an enhancer element within the intron sequence, acting in an orientation-independent manner.

Moreover, introns are absolutely required for the expression of the Chlamydomonas *psaF* gene and expression of its cDNA has never been achieved (Hippler, Stevens and Rochaix, unpublished results). These examples highlight the importance of introns in nuclear gene expression.

Several selectable markers for nuclear transformation are available and are well expressed when fused to the strong promoter of the Chlamydomonas small subunit of the ribulosebiphosphate carboxylase/oxygenase gene (*rbcS*). This promoter has also been used to express foreign genes and foreign or endogenous cDNAs, but with only limited success.

It therefore appears that nuclear expression of foreign genes and cDNAs in Chlamydomonas is particularly difficult with existing expression vectors. This situation limits both the study of new genes often isolated as cDNAs, the manipulation of the nuclear genome, and the use of Chlamydomonas as expression host for production of recombinant proteins.

It is an object of the present invention to provide an efficient expression system for endogenous and foreign genes and cDNAs for use in unicellular green algae such as Chlamydomonas, and in other photosynthetic organisms such as higher plants

This objective is achieved by use of the Chlamydomonas *psaD* transcription regulatory regions, particulary the promoter region, and functional fragments thereof.

The Chlamydomonas nuclear *psaD* gene is highly expressed and encodes an abundant 20 KDa subunit of the photosystem I (PSI) complex. PsaD is required for the binding of PsaC and PsaE to the PSI complex, and furthermore appears to be the Fd docking protein of PSI. This sub-unit is essential for the in vivo function of PSI. The cDNA encoding the Chlamydomonas nuclear PpsaD subunit has recently been cloned and sequenced (Farah et al, 1995).

In the framework of the present invention, the inventors have cloned and characterised the Chlamydomonas *psaD* gene. The structure of the gene has been found to be unusual, as its coding sequence does not contain any introns. The inventors therefore postulated that all sequences required for its strong expression may be located in the surrounding non-translated regions, and that this situation would allow the replacement of the *psaD* coding sequence by another gene of interest without loosing important regulatory sequences.

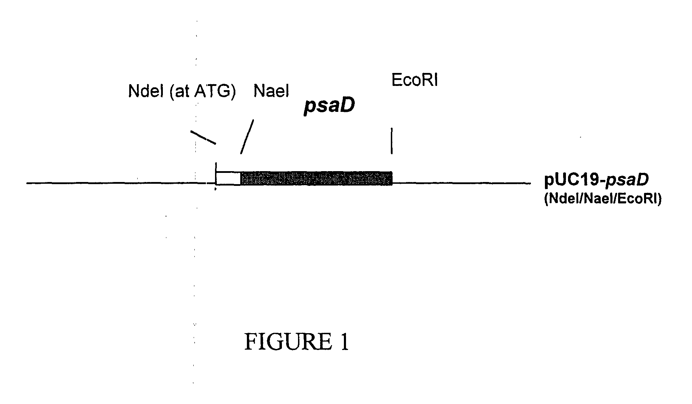

The inventors have cloned and sequenced a 2Kb fragment of nuclear genomic DNA containing the *psaD* gene (Figure 1). The 2kb fragment comprises the 5' untranslated region, including the sequences responsible for initiation of transcription, the coding region, and the 3' untranslated region. Unique restriction sites have been introduced allowing the easy replacement of the *psaD* coding sequence by other sequences of interest. This system can be used to successfully express different endogenous and foreign cDNAs. Furthermore, as the *psaD* gene product is imported into the chloroplast in a post-translational manner, a site has been engineered immediately after the chloroplast transit peptide to allow targeting of the expressed protein to the chloroplast.

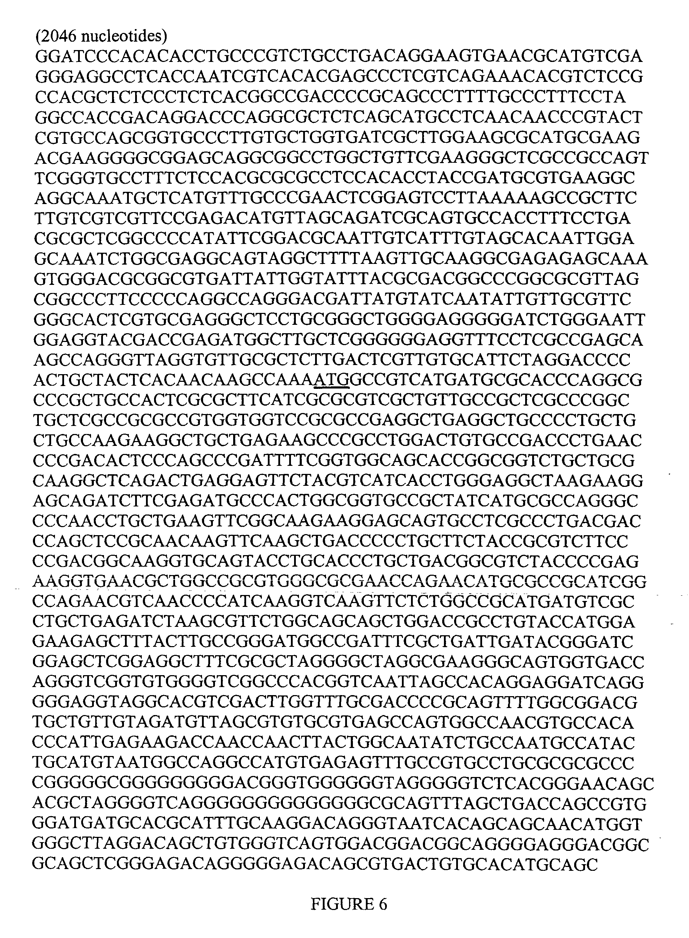

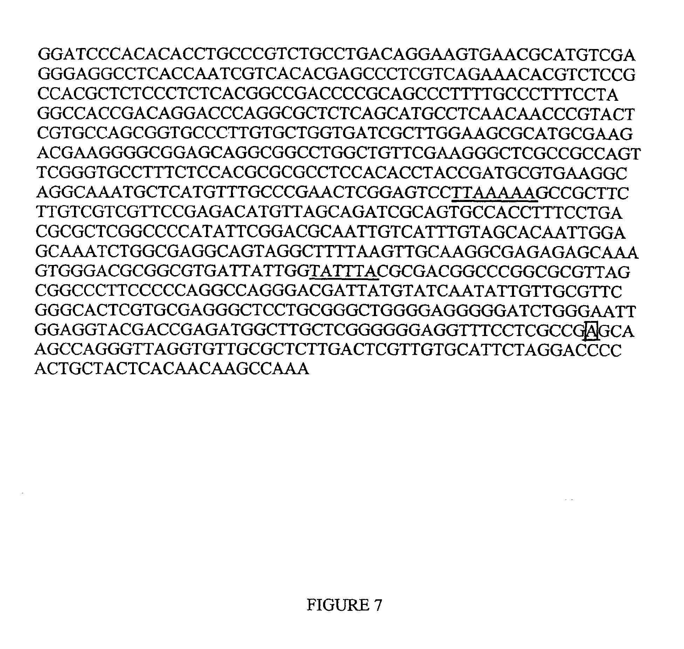

The 5' transcription regulatory element of the psaD gene is shown in Figure 7, corresponding to nucleotides 1 to 823 of the full gene sequence illustrated in Figure 6.

In the context of the present invention, the following terms are defined in the following manner :
- *transcription regulatory element* : any element within a nucleic acid sequence which has the capacity, alone or in association with other cellular components, to initiate, enhance, repress, induce or terminate transcription of genomic DNA into RNA. An element of the invention may exhibit one or more of these capacities. Generally speaking, the genomic DNA whose transcription is regulated by the element is positioned in proximity to the element, either 5' or 3'. It is however possible that the elements act *in trans* on distal sequences. It is also possible that the element(s) act in association with other distal control sequences, or cellular components such as proteins. The term « transcription regulatory element » includes promoters (transcription initiation regions), orientation-dependent or -independent enhancers, repressors, and transcription termination signals. The elements may be situated upstream or downstream of the transcription initiation site, depending upon their precise function.
- *promoter* : transcription initiation region. This region comprises sequence elements necessary for the inititation of transcription by the appropriate RNA polymerase. Generally, this region is situated upstream of the coding region, 5' to the transcription initiation site.
- *gene* : nucleic acid molecule coding for a functional polypeptide or RNA molecule, together with associated transcription regulatory sequences. The gene may further comprise non-coding sequences. Introns may be present.
- *coding region* : region within a gene coding for a functional polypeptide or RNA molecule.
- *chimeric gene* : gene comprising sub-parts of different origin, for example, a coding region originating from a higher plant gene, and transcription regulatory signals originating from an algal gene.
- *heterologous* : two entities are heterologous with respect to each other when they do not naturally occur in association with each other. For example, two nucleic acid sequences are said to be heterologous with respect to each other when the sequences do not originate from the same gene within a given organism, or from the same organism. A gene or part of a gene is said to be heterologous with respect to an organism when the said gene or part of a gene does not naturally occur within that organism.
- % *nucleic acid identity* : indicates the degree of identity between two nucleic acid sequences over the entire length of the sequences. If the sequences under consideration are of different length, % identity is expressed with respect to the entire length of the shortest sequence. To calculate the % identity, two sequences are aligned so as to maximise matches of identical bases (gaps of upto can be tolerated), and the number of identical bases is divided by the total length of the shortest sequence.
- % identity between two polynucleotide sequences can be determined conventionally using known computer programs such as WU-BLAST-2.0a19 (Altschul et al., J. Mol. Biol., 1997, 215 : 403-410 ; http://blast.wusti.edu/blast/README.html, (referred to generally as « BLAST ») ; the GAP program in www.gcg.com ; the ALIGN program found in the FASTA version 1.7 suite of sequence comparison programs (Pearson et al., 1988 ; Pearson 1990 ; program available from William R. Pearson, Department of Biological Chemistry, Box 440, Jordan Hail, Charlottesville, Va.) ; the BESTFIT program (Wisconsin Sequence Analysis Package, Version 8 for Unix, Genetics Computer Group, University Research Park, 575 Science Drive, Madison, Wis, 53711). BESTFIT uses the local homology algorithm of Smith and Waterman, Adv. Appl. Math., 2 : 482-489, 1981, to find the best segment of homology between two sequences.
- *fragment* : a part of an entity, not including the entire entity. For example, a fragment of a nucleic acid sequence refers to parts or segments of that sequence, but does not include the full length sequence.
- *functional fragment :* a fragment which conserves all or part of the function of the entity from which it is derived.

The invention will now be described in detail.

A first aspect of the present invention concerns the Chlamydomonas *psaD* transcription regulatory regions per se. More particularly, the present invention relates to an isolated nucleic acid molecule comprising a transcription regulatory element, characterised in that it consists of, or comprises :
i) the nucleic acid sequence illustrated in Figure 7, or
ii) a functional fragment of the nucleic acid sequence illustrated in Figure 7, or
iii) a sequence having at least 70% identity, for example at least 80% or 85% identity with the sequence of Figure 7.

The transcriptional regulatory elements of the invention, which will be refered to hereafter by reference to elements (i), (ii) and (iii) as defined above, are usually of less than 15kb in size, and preferably less than about 10kb or 5kb, and permit the initiation, enhancement, repression, or induction of transcription of coding sequences, placed under their control, in *Chlamydomonas reinhardtii*. Depending upon the particular sequence in question, they may also be functional in the nuclear genome of other photosynthetic organisms belonging to the phylogenetic kingdom *Planta,* particularly higher plants and other unicellular green algae such as *Chlorella,* and *Volvox*.

More particularly, element (i) exhibits a transcription initiation function, or « promoter » function, with respect to any coding sequence placed under its control.

Derivatives (ii) and (iii) may have a transcription initiation function, and / or, depending on the precise structure of the derivative in question, may have an enhancer or repressor function. Such enhancer or repressor functions can be used in association with other transcription initiation regions or promoters, to further modulate regulation of transcription.

The transcription initiation regions are particularly preferred embodiments of the invention. These regions give rise to high, constitutive expression of endogenous and heterologous genes, and cDNA. They are thus entirely functional in association with coding sequences devoid of introns. Expression levels obtained with the psaD promoter vary depending upon the coding sequence used. However, generally speaking, higher expression levels than those obtained with the RbcS2 promoter are obtained, with respect to the same coding sequence. Improvements from twofold upto infinite over the RbcS2 promoter have been observed, as some cDNAs could never be expressed in the RbcS2 context, e.g. the psAF cDNA.

The sequence depicted in Figure 7 corresponds to the region immediately 5' to the psaD coding sequence in the nuclear genome of *Chlamydomonas reinhardtii*. The entire sequence illustrated in Figure 7 may be used as a transcription initiation element, or alternatively, fragments of the illustrated sequence may be used.

Such transcription initiation fragments normally consist of, or comprise, at least 25, preferably at least 50, more preferably at least 100, even more preferably at least 200 and most preferably at least 300 consecutive nucleotides of the nucleic acid sequence illustrated in Figure 7. Typical lengths of fragments which are active as transcriptional initiation regions are between 200 and 600 nucleotides. Particularly preferred fragments of this variant of the invention are sequences which correspond to the Figure 7 sequence after undergoing a 5' deletion of upto 500 nucleotides, for example, 5' deletions of from 1 to 450 nucleotides, particularly, from 10 to 200 nucleotides.

Alternatively, or in addition to the 5' deletions, 3' deletions of upto approximately 80 or 100 nucleotides, for example upto 20, 30, 40, 50, 60, 70 or 77 nucleotides with respect to the Figure 7 sequence can be made, whilst conserving transcriptional initiation activity. This region corresponds to the 5'UTR region of the gene. Normally, to avoid abolition of the « promoter » activity, the 3' deletions with respect to the Figure 7 sequence should not go beyond the transcription initiation site of the psaD gene, which, according to preliminary primer extension analysis as well as amplification of the 5' UTR regions of psaD from cDNA libraries, is situated at position 747 (-77 relative to the ATG illustrated in Figure 6). The functional fragments of the invention usually comprise sequences found to be essential in other polymerase II promoters, for example one or more TATA boxes.Two putative TATA box-containing promoters have been identified around positions 574 and 386 of the sequences illustrated in Figures 6 and 7.

Particularly preferred fragments of the sequence in Figure 7 are fragments comprising approximately bases 350 to 750, or 550 to 750, or 350 to 823 (± 1 or 2) or 550 to 823 (± 1 or 2).

Fragments of the Figure 7 sequence which exhibit transcriptional regulatory activity other than initiation activity, for example repressor or enhancer activity, may include the 5' extremity of the Figure 7 sequence, for example fragments including at least the first 25 nucleotides at the 5' extremity of the illustrated sequence, and preferably at least the first 50 nucleotides. This segment of the Figure 7 sequence may exhibit repressor activity.
Enhancer or repressor elements of the invention usually have a total length of at least 25 nucleotides, upto around 300 nucleotides, for example 200 to 250 nucleotides.

The capacity of the different elements and fragments to regulate transcription, particularly to initiate transcription, can be tested by construction of a chimeric gene, comprising the coding region of a selectable marker or reporter, operably linked to a DNA sequence as defined in (i), (ii) or (iii) above, followed by the transformation of a cell of interest, preferably C. reinhardtii, with a construct comprising the chimeric gene. Expression of the selectable marker gene or reporter is detected either at the protein level by Western blot for example, or at the RNA level by Northern blot. Detection of expression of the RNA or protein indicates a transcriptional initiation activity.

If appropriate, in order to detect other types of transcriptional regulation such as enhancement or repression, the level of transcription obtained using the fragment under test can be compared to the level of transcription obtained using the full length sequence of Figure 7, or to the level obtained using other fragments of this sequence. This comparison enables the detecion of enhancer or repressor activity which may be associated with particular segments of the transcription regulatory sequence illustrated in Figure 7.

The transcription regulatory sequences of the invention also include sequences having at least 70% identity, for example at least 80% or at least 85% or at least 90% identity, and preferably at least 95% or at least 97% identity, with the nucleic acid sequence illustrated in Figure 7. These sequences are typically isolated from strains of *Chlamydomonas reinhardtii* different from the particular one used to isolate the psaD gene illustrated in Figure 6 (wild-type strain 137c, available from the Chlamydomonas Culture Collection at Duke University, North Carolina, USA), but may also comprise mutants or synthetic variants of the sequence illustrated in Figure 7, provided that the transcription regulatory activity is conserved.

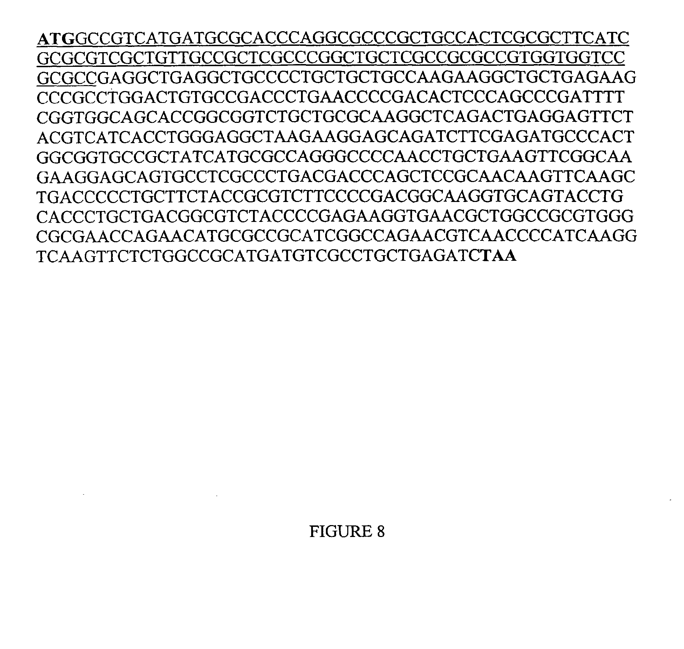

The transcription regulatory elements of the invention may be obtained by chemical synthesis of approriate DNA segments on the basis of the sequence illustrated in Figure 7. Alternatively, the elements may be isolated from genomic nuclear DNA of Chlamydomonas reinhardtii, using techniques such as hybridisation of probes specific for the psaD coding sequence, as described in the Examples below. Probes, having lengths from around 50 to around 500 bases, for example from 100 to 250 bases, usually are capable of hybridising, in stringent conditions, to parts of the coding sequence illustrated in Figure 8. Generally speaking, the DNA segments of interest as regulatory elements are situated upstream of the ATG translation initiation codon of the psaD gene, the 3' extremity of the regulatory element being commonly within approximately 200 bases upstream of the ATG codon, although sequences having enhancer or repressor function may be further upstream, for example 700 to 800 bases upstream of the ATG codon. Segments having transcription initiation function typically have a length of between about 300 and 1500 bases.

The transcriptional regulatory elements of the invention may be introduced into a suitable eukaryotic cell such as a green alga or a higher plant in several different forms. For example, the elements may be introduced in an appropriate vector, independently of an associated exogenous coding region when replacement or complementation of endogenous transcriptional control sequences is desired. Such an application normally requires that the element be flanked, in the vector, by regions of homology with the genome of the host cell, corresponding to regions flanking the desired insertion site. The transcription regulatory element is then integrated into the host genome in a targeted manner by homologous recombination. Such a technique enables the expression of endogenous coding sequences under the control of the psaD regulatory sequences.

Alternatively, the transcription regulatory elements may be introduced into a suitable host cell in the form of a chimeric gene, so as to obtain expression of an exogenous coding sequence under the control of the psaD regulatory sequences.

The invention also concerns these chimeric genes, comprising transcription regulatory sequences derived from the nuclear Chlamydomonas psaD gene, operably linked to at least one coding region. In particular, the chimeric genes are characterised in that the transcription regulatory sequences comprise at least one element (i), (ii) or (iii), as described above, and a coding region which is heterologous with respect to the said transcription regulatory element.

Generally speaking, in the chimeric genes of the invention, the transcription regulatory element (i), (ii) or (iii) as defined above, is positioned 5' to the heterologous coding sequence. An exception to this principle may occur when the regulatory element is an enhancer, in which case, the element may be 5' or 3' with respect to the coding sequence.

The chimeric genes of the invention may comprise more than one transcription regulatory element (i), (ii) and/or (iii) described above, or alternatively may comprise one regulatory element of the invention in association with a conventional regulatory signal. For example, two « promoter » regions of the invention may be juxtaposed together to provide a « double promoter » effect. A traditional enhancer element may also be used in conjunction with the transcription initiation elements of the present invention. Furthermore, regulatory sequences for induced gene expression, such as light, metal or drug responsive elements, may be combined with the psaD regulatory sequences to confer an inducible character.

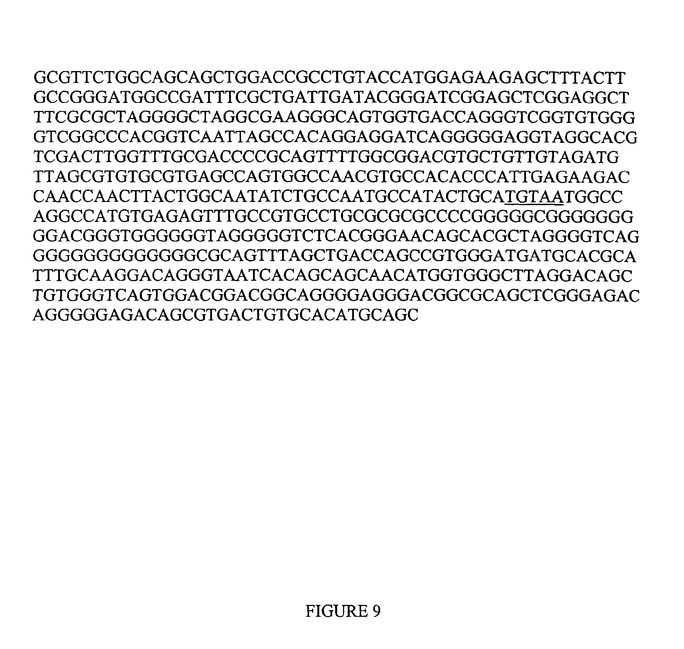

The chimeric gene further contains transcriptional termination signals, positioned 3' to the coding sequence, for example transcription termination signals of Chlamydomonas reinhardtii origin. Preferably, the transcription termination signals are also derived from the psaD gene of Chlamydomonas reinhardtii, and comprise all or part of the sequence illustrated in Figure 9.

Particularly preferred chimeric gene structures according to the invention result from the in-frame insertion of a heterologous coding sequence either at the translation initiation codon ATG of the psaD gene, or, since the psaD gene comprises a chloroplast transit peptide, at the site corresponding to the 3' end of the chloroplast transit encoding region (which is underlined in Figure 8), if targeting of the chloroplast is desired.

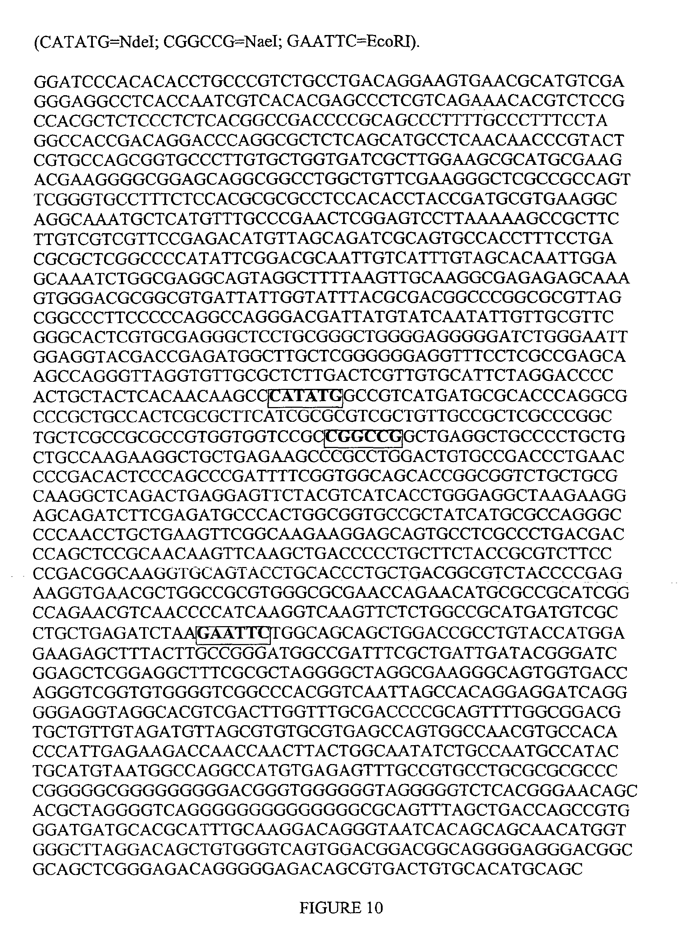

With respect to the sequences illustrated in Figures 6 and 10, these structures correspond to an insertion between the EcoRI site and either the NdeI site, or the NaeI site of the psaD gene (as illustrated in Figure 10), the endogenous coding region being replaced by the heterologous sequence. Additional restriction sites may be introduced for the easy introduction of Tag sequences at the N-terminus and C-terminus of the protein.

As far as the heterologous coding region is concerned, any coding region of interest may be used. This part of the chimeric gene is heterologous with respect to the transcription initiation region, and may be also heterologous with respect to the organism to be transformed. Alternatively, the coding region may be endogenous to the organism to be transformed, for example C. reinhardtii.

The coding region may comprise cDNA, and in this case is therefore devoid of introns, or alternatively may comprise genomic DNA with or without naturally associated introns. Preferred coding regions are free of naturally associated introns. However, further improvement of expression may be obtained by the artificial addition of introns into the 5' and/or 3' unstranslated regions of the chimeric gene, for example intron 1 of the Chlamydomonas rbcs2 gene.

Examples of suitable coding regions for use in the chimeric genes of the invention include the coding regions of selective marker genes such as Arg, a Chlamydomonas gene encoding argininosuccinate lyase ; Ble, a bacterial gene encoding resistance to phleomycin and zeomycin ; reporter genes of prokaryotic or eukaryotic origin ; therapeutically active proteins such as interferons, hormones, growth factors, insulin, immunogens, erythropoietin, tPA, proteins affecting metabolism ; proteins conferring resistance to the expressing organism, such as insecticides, herbicides, viral resistance etc.

The invention further relates to organisms stably transformed in the nuclear genome by the transcription regulatory sequences (i), (ii) and (iii), or by the chimeric genes defined above. In particular, the invention encompasses eukaryotic cells from an organism belonging to the phylogenetic kingdom *Planta,* particulary a cell of a photosynthetic organism such as green alga, or higher plants.

As examples of green algae, *Chlamydomonas, Chlorella,* and *Volvox* can be cited. Particulary preferred green algae are cells of *Chlamydomonas reinhardtii*. Indeed, the regulatory sequences of the invention being of Chlamydomonas origin, they have been found to be particularly efficient in this species.

As examples of higher plants, angiosperms and gymnosperms can be cited, including monocotyledonous and dicotyledonous plants, for example plants belonging to the families *Brassicae, Cucurbitacae, Solanacae, Compositae*, *Graminae* etc.

The transformation processes employed to introduce the transcription regulatory elements of the invention into eukaryotic cells are those typically used in the art.

For Chlamydomonas the nuclear transformation techniques include biolistic delivery, electroporation, agitation with glass beads, or use of silicon carbide whiskers. A particularly preferred technique is that described by Kindle et al. (1990) are suitable.

For higher plants, conventional transformation methods are also used, for example, direct DNA transfer using biolistic delivery, electroporation, Agrobacterium-independent Ti plasmid transformation or Agrobacterium-mediated transformation.

The invention also relates to the transgenic plants produced by transformation of plant cells with the psaD transcription regulatory sequences, and regeneration of transformants. The transgenic plants have the capacity to express an endogenous or exogenous coding sequence, in at least a portion of their cells, during at least a portion of their development, under the control of the psaD-derived regulatory elements.

In a further embodiment, the invention relates to a process for the production of heterologous proteins in a eukaryotic cell, said proteins being expressed under the control of the psaD regulatory elements. According to this process, a chimeric gene of the invention, whose coding region encodes a protein heterologous to the cell is transformed as described above, and the cell is then subjected to conditions permitting the stable expression of the chimeric gene. Optionally, the heterologous protein may be recovered from the cell or its supernatant, using conventional techniques. Such a process may be applied to cells in culture in vitro, particularly to green algae such as *Chlamydomonas reinhardtii*, or alternatively, to whole plants.

When the process of the invention is used for producing recombinant proteins in plants, the basic process of transformation further comprises the steps of regeneration of a transgenic plant from the transformed cell containing the chimeric gene, and multiplication of the thus obtained transgenic plant to obtain transgenic progeny. The transgenic progeny are also part of the present invention. The expressed heterologous protein may be recovered from the plant tissue, or alternatively may be left in the plant tissue, depending upon the subsequent use of the protein.

In a further embodiment, the invention also relates to nucleic acid molecules comprising the full Chlamydomonas rheinhardtii gene, comprising 5' and 3' transriptional regulatory elements and coding sequences. Such a nucleic acid is illustrated in Figure 6. Alternatively, the gene may comprise a sequence having at least 90% and preferably at least 95% identity with said sequence.

Particular aspects of the invention are illustrated in the figures :
Figure 1. Schematic representation of the *psaD* genomic fragment. The introduced restriction sites are indicated. The chloroplast transit peptide is indicated by an open box.
Figures 2A and 2B. Transformation efficiency of the *Arg* and *Ble* genes expressed under the control of different promoters.
Figure 3. Western blot analysis of total cells extracts probed with an antibody raised against the Ble protein. Equal mounts of total protein were loaded per lane.
Figure 4. Western blot analysis of total cells extracts from WT, 3bF and transformants obtained by co-transformation with pSP115 and *pKS-D-PsaF.* The blot was incubated with antibodies raised against the PsaA and PsaF proteins. The growth phenotype on minimal medium and high light conditions is indicated at the bottom of the figure.
Figure 5. western blot analysis of total cells extracts (T) or isolated chloroplasts (C) from different strains. The antibodies used are indicated on the left and capacity to grow on medium containing 5*µ*g/ml zeomycin is shown at the bottom of the figure.
Figure 6. Genomic fragment containing the *psaD* gene of *Chlamydomonas reinhardtii*. The ATG translation start site is underlined.
Figure 7. PsaD transcription regulatory region and 5'UTR. The putative transcription start site (boxed) is located at position 747 (-77 relative to the ATG). Two putative TATA boxes are identified around positions 574 and 386 (underlined).
Figure 8. PsaD coding sequence (Initiation and stop codons are shown in bold, chloroplast transit peptide is underlined)
Figure 9. PsaD 3'UTR (The putative polyadenylation signal is underlined)
   Figure 10. Same sequence as in Figure 6 with the unique restriction sites introduced by site-directed mutagenesis shown in bold and boxed.
   (CATATG=NdeI; CGGCCG=NaeI; GAATTC=EcoRI).

### EXAMPLES

### I. Cloning and characterisation of the psaD gene of Chlamydomonas reinhadrdtii.

### Procedure

We first screened a cosmid library containing *Chlamydomonas reinhardtii* genomic DNA fragments with a probe specific for the *psaD* coding sequence derived from the previously described cDNA of *psaD* (Farah et al, 1995). This library contains *MboI* fragments of 30-40 Kb obtained by partial digest of total genomic DNA from *C*. *reinhardtii* (strain *cw15* mt-) The fragments were cloned at a *BamHI* site into the pPR691 cosmid vector (Jiang et al., 1987).

After identification of positive clones, a 2 Kb *BamHI/ApaLI* fragment containing the *psaD* gene was subcloned into the pKS vector (Stratagene) at the *BamHI/EcoRV* sites. This fragment was fully sequenced on both strands. It contains a 591 bp open reading frame encoding the PsaD protein. Comparison of the *psaD* genomic sequence with that of the cDNA, indicates that there are no introns in the coding sequence.

In order to intoduce convenient cloning sites at the ATG initiation codon, after the chloroplast transit peptide and after the stop codon, the genomic fragment was cloned at the *XbaI/HindIII* sites of a pUC19 vector in which the *EcoRI/NdeI* fragment has been removed. Three unique restriction sites were introduced by site-directed mutagenesis : a *NdeI* site at the ATG ; a *NaeI* site at the end of the chloroplast transit peptide ; an *EcoRI* site after the TAA stop codon. These sites allow one to easily replace the *psaD* coding sequence with other DNA fragments at the endogenous ATG codon or as translation fusions with the chloroplast transit peptide for chloroplast protein targeting.

### II. Nuclear gene expression

### 1. Selectable markers

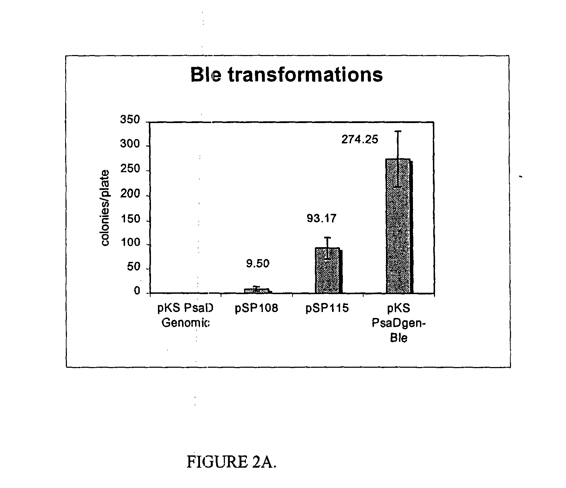

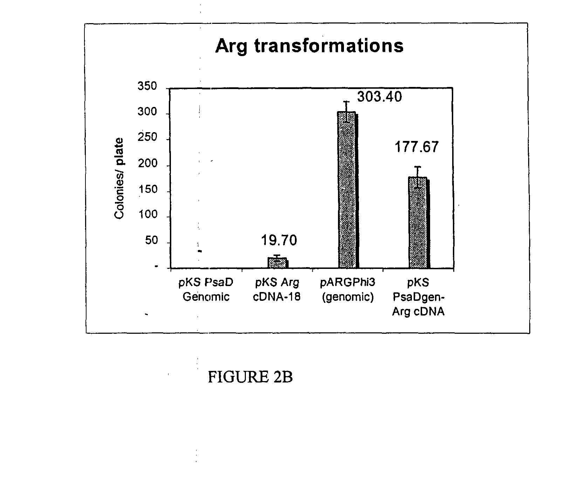

The capacity of the *psaD* promoter to drive the expression of other genes has first been tested with selectable markers such as *Arg* (an endogenous gene) which encodes an argininosuccinate lyase and can complement mutants deficient in arginine biosynthesis and with the *Ble* marker (a bacterial gene) which confers resistance to phleomycin and zeomycin. The *Arg* DNA and the *Ble* gene were cloned into the pUC19-*psaD* (or *pKS-psaD)* vector and their transformation efficiency compared to other transforming DNAs (Figure 2) .

The results clearly indicate that about 25-fold more transformants are obtained when the expression of *Ble* is driven by the *psaD* promoter, compared to the *rbcS* promoter (vector pSP108). Furthermore, the *psaD* promoter gives 3-fold better results than the pSP115 vector which contains an *rbcS* intron in the *Ble* coding sequence to enhance its expression (Lumbreras et al., 1998). The *psaD* promoter is also able to drive efficiently the expression of the *Arg* gene although not at the same level as the full genomic *Arg* fragment of 7.8 Kb.

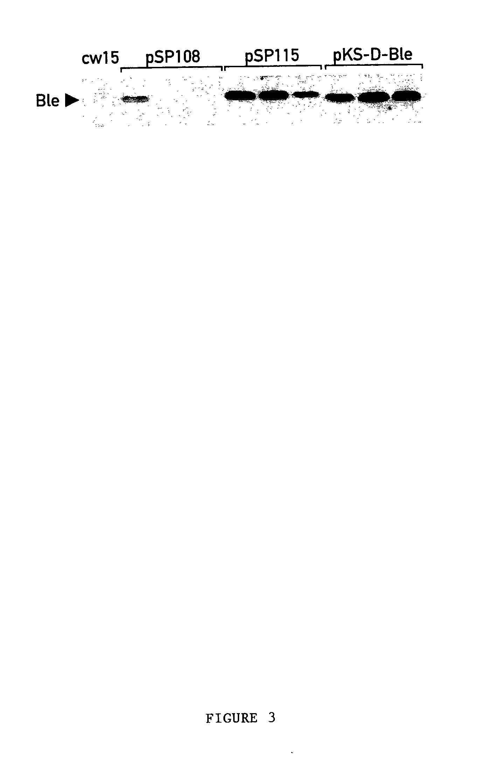

The strength of the *psaD* promoter was further studied by western blot analysis of total cells extracts from transformants obtained with the different *Ble* expression constructs (Figure 3).

The accumulation of Ble protein in the transformants obtained with the *psaD* expression vector is clearly higher than in the transformants obtained with the pSP108 and pSP115 constructs. This result indicates that the higher transformation efficiency observed with the *psaD*-driven constructs is due to higher expression levels of the *Ble* gene.

### 2. Expression of endogenous genes

We have also tested the expression of endogenous genes for which no selection can be applied, by performing co-transformation experiments.

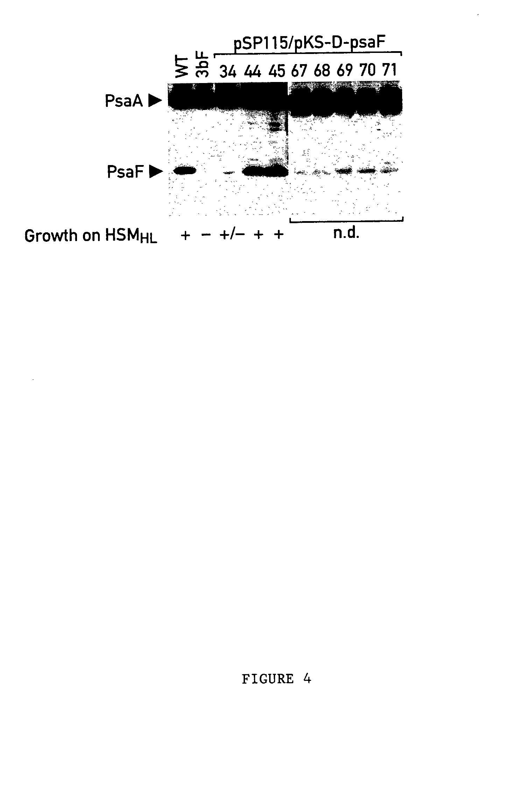

The nuclear *psaF* gene contains introns that are required for expression and attempts to express the *psaF* cDNA from the *rbcS* promoter have been unsuccessful (Hippler, Stevens and Rochaix, unpublished results). In order to test the capacity of the *psaD* promoter to express cDNAs which could not be expressed so far, we introduced the *psaF* coding sequence into the *pKS-psaD* expression vector and transformed the *Chlamydomonas* 3bF strain which is deficient for the psaF gene (Farah et al., 1995). The *pKS-D-psaF* construct was co-transformed with the pSP115 plasmid. The transformants were selected on zeomycin-containing medium and screened by western blot analysis for the expression of the PsaF protein (Figure 4).

In the case of the transformants 44 and 45, the PsaF protein accumulates at levels comparable to the wild-type strain, indicating that the cDNA is efficiently expressed. A number of other transformants accumulating intermediate levels of PsaF could also be identified. The PsaF protein is required for photoautotrophic growth under high light conditions. Interestingly the growth capacity under these conditions correlated with the level of psaF expression, indicating that the expressed protein is functional (Figure 4). These results show that the *psaD* promoter is able to drive the expression of a cDNA that was difficult to express with other vectors.

Another example of endogenous cDNA expression is provided by the successful expression of a HA-tagged version of the MAA2 cDNA (Perron et al., 1999; Perron and Rochaix, unpublished results). Previous attempts to detect the tagged protein expressed in *Chlamydomonas,* had been unsuccessful whereas it could be easily detected in the transformants obtained with the *psaD-MAA2-HA* construct (data not shown).

### 3. Protein targeting to the chloroplast

We have taken advantage of the chloroplast transit peptide located at the N-terminus of the PsaD protein to target expressed proteins to the chloroplast of *Chlamydomonas*.

The *Ble* gene was fused to the transit peptide and was either transformed alone or co-transformed with a plasmid containing the *Arg* gene into a strain deficient for the argininosuccinate lyase. The transformants were selected either for growth on zeomycin or on medium lacking arginine, in the case of co-transformation experiments. The growing colonies were then screened by western blot analysis for expression of the Ble protein. Chloroplasts of four selected strains were isolated : cw15, the starting cell-wall deficient strain; pArg/pUC19D-Ble, a strain obtained by co-transformation with pArg and the chloroplast-targeting construct for Ble; pUC19D-Ble, a strain obtained by transformation with the chloroplast-targeting construct and direct selection on zeomycin; pKSD-Ble, contains the Ble expression construct without chloroplast targeting.

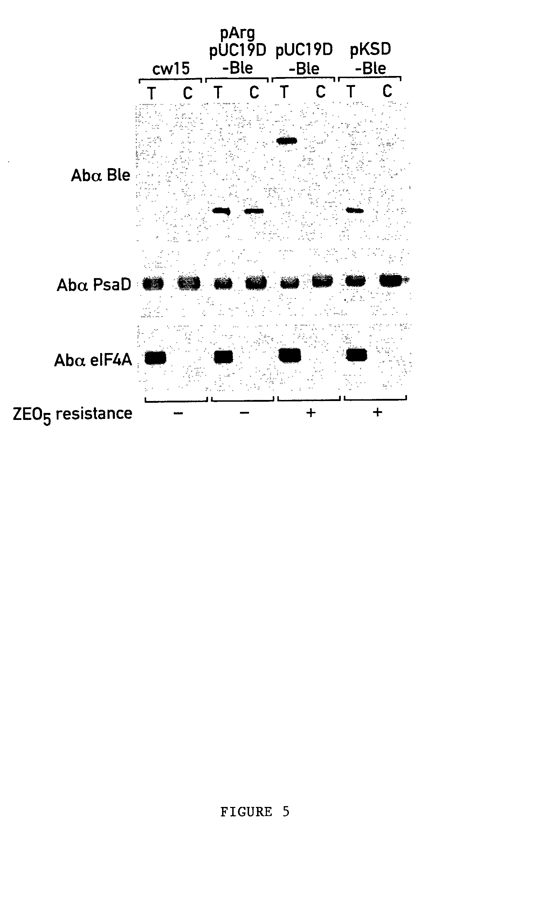

Western blot analysis was performed on total cells extracts as well as on isolated chloroplast of these four strains (Figure 5). The blot was incubated with antibodies raised against eIF4A (a cytosolic marker), PsaD (a chloroplast protein) and Ble. The signal corresponding to eIF4A is only observed in the total extracts, indicating that there is no cytosolic contamination in the chloroplast preparations. The PsaD signal is used as a loading control. The signal corresponding to the Ble protein is observed in the total cell extracts of all strains containing Ble-expressing constructs. However, the Ble protein is only present in the chloroplast of the pUC19D-Ble strain that has been co-transformed with the pArg plasmid and therefore not selected for zeomycin resistance. Interestingly, this strain is sensitive to zeomycin although it expresses Ble, suggesting that when Ble is targeted to the chloroplast, it can no longer confer resistance to the drug. In line with this observation, the size of the Ble protein is larger in the strain containing the pUC19D-Ble construct, but that has been selected for zeomycin resistance. This suggests that an integration event has occurred that lead to the fusion between Ble and another protein. This fusion protein cannot be imported into the chloroplast and therefore confers resistance to zeomycin.

Taken together, these experiments show that the expression construct that we have designed can be used to target the expressed protein to the chloroplast. This possibility is a major advantage for the study and engineering of processes taking place into the chloroplast .

### References

**Day et al**, (1990), Physiol Plant 78 : 254-260

**Blankenship and Kindle**, (1992), Mol Cell Biol 12 : 5268-5279

**Farah J, Rappaport F, Choquet Y, Joliot P, Rochaix JD** (1995) EMBO J. 14(20):4976-84

**Farah et al.** (1995) Plant Physiol 107(4):1485-6

**Jiang et al**, (1987) Plasmid 18: 170-172

**Lumbreras V, Stevens D, Purton S** (1998) Plant J 14(4), 441-447

**Kindle K.L**, (1990) Proc. Natl. Acad. Sci. USA, 87 : 1228-1232

**Kindle and Sodeinde**, (1994) J. Appl. Phycol 6 : 231-238

**Rochaix JD** (1995) Annu. Rev. Genet. 29:209-30

**Silflow C**. (1998) *In The Molecular Biology of Chloroplast and Mitochondria in Chlamydomonas* pp 25-40

## Claims

**1.** Transcription regulatory element, characterised in that it comprises :
i) the nucleic acid sequence illustrated in Figure 7, or
ii) a fragment thereof, or
iii) a sequence having at least 70% identity with the sequence of Figure 7.

**2.** Transcription regulatory element according to claim 1 characterised in that said fragment comprises at least 25 consecutive nucleotides, for example at least 50 consecutive nucleotides, of the nucleic acid sequence illustrated in Figure 7.

**3.** Transcription regulatory element according to claim 1 characterised in that it comprises a sequence having at least 80 and preferably at least 85% identity with the nucleic acid sequence illustrated in Figure 7.

**4.** Chimeric gene comprising transcription regulatory sequences and at least one coding region, characterised in that the transcription regulatory sequences comprise at least one element according to any one of claims 1 to 3, said coding region being heterologous with respect to said transcription regulatory element.

**5.** Chimeric gene according to claim 4, characterised in that the coding region is free of introns naturally associated with said coding region.

**6.** Chimeric gene according to claim 4 or 5, characterised in that said coding region comprises cDNA.

**7.** Chimeric gene according to any one of claim 4 to 6 characterised in that the coding region comprises a coding sequence which is heterologous to Chlamydomonas reinhardtii.

**8.** Chimeric gene according to any one of claims 4 to 7 characterised in that said transcription regulatory sequences further comprise transcription termination signals, positioned 3' to the said coding sequence.

**9.** Chimeric gene according to claim 8 characterised in that said transcription termination signals are of Chlamydomonas reinhardtii origin.

**10.** Chimeric gene according to claim 9 characterised in that said transcription termination signals are from the psaD gene of Chlamydomonas reinhardtii.

**11.** Cell stably transformed by a chimeric gene according to any one of claims 4 to 10.

**12.** Cell according to claim 11 wherein said cell is a eukaryotic cell.

**13.** Cell according to claim 12 wherein said cell is a cell from an organism belonging to the phylogenetic kingdom *Planta*.

**14.** Cell according to claim 13 which is a cell of a higher plant.

**15.** Cell according to claim 13, which is a cell of a unicellular photosynthetic alga.

**16.** Cell according to claim 15 which is a cell of Chlamydomonas reinhardtii.

**17.** Transgenic plant comprising cells according to claim 14.

**18.** Process for the transformation of a eukaryotic cell, characterised by the introduction into said cell of a chimeric gene according to any one of claims 4 to 10.

**19.** Process for the production of heterologous proteins in a cell, characterised by the introduction into said cell of a chimeric gene according to any one of claims 4 to 10, the coding region of said chimeric gene encoding a protein heterologous to said cell, subjecting the cell to conditions permitting the stable expression of said chimeric gene, and optionally, recovering said heterologous protein from said cell or its supernatant.

**20.** Process according to claim 19 wherein said cell is a unicellular green alga such as Chlamydomonas reinhardtii.

**22.** Process according to claim 21, said process further comprising the steps of regeneration of a transgenic plant from the cell containing the chimeric gene, multiplication of the thus obtained transgenic plant to obtain transgenic progeny, and optionally, recovery of the expressed heterologous protein.

**23.** Use of a transcription regulatory element according to any one of claims 1 to 3 for the expression of a heterologous coding sequence in a eukaryotic cell.

**24.** Use according to claim 23 wherein said eukaryotic cell is a plant cell.

**25.** Use according to claim 19 wherein said eukaryotic cell is a unicellular alga, particularly Chlamydomonas reinhardtii.

**26.** Nucleic acid molecule comprising the psaD gene of *Chlamydomonas reinhardtii*.

**27.** Nucleic acid molecule according to claim 26, wherein said gene comprises the nucleic acid sequence illustrated in Figure 6, or a sequence having at least 70% and preferably at least 80% identity with said sequence.

**27.** Transcription regulatory element characterised in that it is obtainable by isolation, from genomic DNA of *Chlamydomonas reinhardtii,* of a DNA segment situated within 200 bases upstream of the ATG translation initiation codon of the psaD gene, said segment having between 300 and 1500 bases.

**28.** Transcription regulatory element according to claim 27, situated, in the genomic DNA of *Chlamydomonas reinhardtii,* within 200 bases of the ATG translation initiation codon of the psaD coding sequence illustrated in Figure 8.

**29.** Expression vector comprising a chimeric gene according to any one of claims 4 to 10.
